# EUROPÄISCHE PATENTSCHRIFT

(11) **EP 1 054 883 B1**
(45) Veröffentlichungstag und Bekanntmachung des Hinweises auf die Patenterteilung: **17.04.2002**
(21) Anmeldenummer: 99907416.4
(22) Anmeldetag: 26.01.1999
(51) Int. Cl.: C07D 401/12, A01N 43/48

(54) **2-(PYRAZOLYLOXY)-PYRIDIN-3-YLESSIGSÄURE-DERIVATE, SIE ENTHALTENDE MITTEL UND IHRE VERWENDUNG ZUR BEKÄMPFUNG VON SCHADPILZEN UND TIERISCHEN SCHÄDLINGEN**
2-(PYRAZOLYLOXY)-PYRIDIN-3-YLACETIC ACID DERIVATIVES, AGENTS CONTAINING THE SAME AND USE THEREOF AGAINST NOXIOUS FUNGI AND ANIMAL PARASITES
DERIVES DE 2-(PYRAZOLYLOXY)-PYRIDINE-3-YLE-ACIDE ACETIQUE, AGENTS LES CONTENANT ET LEUR UTILISATION POUR LUTTER CONTRE DES CHAMPIGNONS NUISIBLES ET DES PARASITES ANIMAUX

(30) Priorität: 05.02.1998 DE 19804486
(43) Veröffentlichungstag der Anmeldung: 29.11.2000
(73) Patentinhaber: BASF AKTIENGESELLSCHAFT, 67056 Ludwigshafen (DE)
(72) Erfinder: PTOCK, Arne, D-67065 Ludwigshafen (DE); SAUTER, Hubert, D-68305 Mannheim (DE); KIRSTGEN, Reinhard, D-67434 Neustadt (DE); GRAMMENOS, Wassilios, D-67063 Ludwigshafen (DE); GROTE, Thomas, D-67105 Schifferstadt (DE); GYPSER, Andreas, D-68159 Mannheim (DE); BAYER, Herbert, D-68159 Mannheim (DE); GEWEHR, Markus, D-56288 Kastellaun (DE); CULLMANN, Oliver, D-68199 Mannheim (DE); MÜLLER, Bernd, D-67227 Frankenthal (DE); GÖTZ, Roland, D-68809 Neulussheim (DE); RÖHL, Franz, D-67105 Schifferstadt (DE); AMMERMANN, Eberhard, D-64646 Heppenheim (DE); LORENZ, Gisela, D-67434 Neustadt (DE); HARRIES, Volker, D-67227 Frankenthal (DE); STRATHMANN, Siegfried, D-67117 Limburgerhof (DE)
(86) Internationale Anmeldenummer: EP9900470
(87) Internationale Veröffentlichungsnummer: WO9940082

(56) Entgegenhaltungen:
- EP-A- 0 477 631
- EP-A- 0 513 580
- WO-A-95/06033
- WO-A-97/24317
- WO-A-97/24332
- WO-A-97/29093
- WO-A-98/12179
- FR-A- 2 452 493

## Beschreibung

Die vorliegende Erfindung betrifft Verbindungen der allgemeinen Formel I 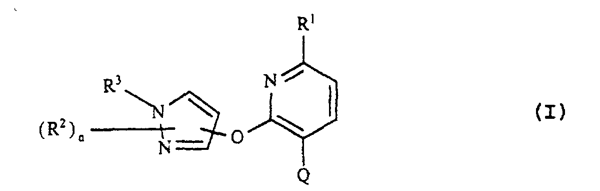 sowie deren Salze, in der die Substituenten und der Index n die folgenden Bedeutungen haben:
- Q: -C(=CHCH₃)-COOCH₃,
-C(=CHOCH₃)-COOCH₃,
-C(=NOCH₃)-COOCH₃ oder
-C(=NOCH₃)-CONH(CH₃);
- R¹: Wasserstoff, Halogen, C₁-C₄-Alkyl, C₁-C₂-Halogenalkyl, C₁-C₄-Alkoxy oder C₁-C₂-Halogenalkoxy;
- R²: Halogen, C₁-C₄-Alkyl, C₁-C₂-Halogenalkyl, C₁-C₄-Alkoxy;
- n: 0, 1 oder 2, wobei die Substituenten R² verschieden sein können, wenn n = 2 ist;
- R³: Phenyl, Pyridyl oder Pyrimidyl, wobei der Phenyl-, Pyridyl- oder Pyrimidylrest einen oder, unabhängig voneinander, zwei oder drei der folgenden Substituenten tragen kann: Halogen, Cyano, C₁-C₄-Alkyl, C₁-C₂-Halogenalkyl, C₁-C₄-Alkoxy, C₁-C₂-Halogenalkoxy oder C₁-C₄-Alkoxycarbonyl.

In der Patentschrift EP-A 579 071 werden allgemein Hetarylessigsäuren, insbesondere diejenigen mit einem 5-Ring-Hetarylrest, als Pflanzenschutzmittel beschrieben. Weiterhin sind in 2-Stellung substituierte Pyridin-3-ylessigsäure-Derivate mit fungizider Wirkung in der WO-A 95/06033 und der WO-A 98/12179 genannt. In der Anmeldung WO-A 98/12179 wird zudem von einer Wirkung der beanspruchten Verbindungen gegen tierische Schädlinge gesprochen.

Schließlich sind aus der WO-A 97/24332 Phenylessigsäure-Derivate mit einem Pyrazolyloxy-Substituenten in ortho-Stellung zur Essigsäuregruppierung bekannt, die eine Wirkung gegen Schadpilze und tierische Schädlinge aufweisen.

Hinsichtlich der Wirkungsspektren und Aufwandmengen können die bekannten Verbindungen dieses Typs noch nicht befriedigen.

Aufgabe der vorliegenden Erfindung war es demnach, verbesserte Wirkstoffe gegen Schadpilze und tierische Schädlinge aufzufinden.

Demgemäß wurden die eingangs definierten Verbindungen I und deren Salze gefunden.

Des weiteren wurden Mittel zur Bekämpfung von Schadpilzen und tierischen Schädlingen, welche die Verbindungen I oder deren Salze enthalten und die Verwendung der Verbindungen I und ihrer Salze und der sie enthaltenden Mittel hierzu gefunden.

Die Verbindungen I sind auf verschiedenen Wegen nach an sich bekannten Verfahren erhältlich. Grundsätzlich ist es bei ihrer Synthese unerheblich, ob zunächst der Pyrazolyloxyrest in 2-Stellung oder die Gruppierung "Q" in 3-Stellung am Pyridinring aufgebaut wird.

Die Verbindungen I sind beispielsweise über die Verbindungen Z erhältlich (Syntheseweg 1), welche ihrerseits durch Umsetzung von Verbindungen der Formel II mit Hydroxypyrazolen der Formel III zugänglich sind.

### Syntheseweg 1:

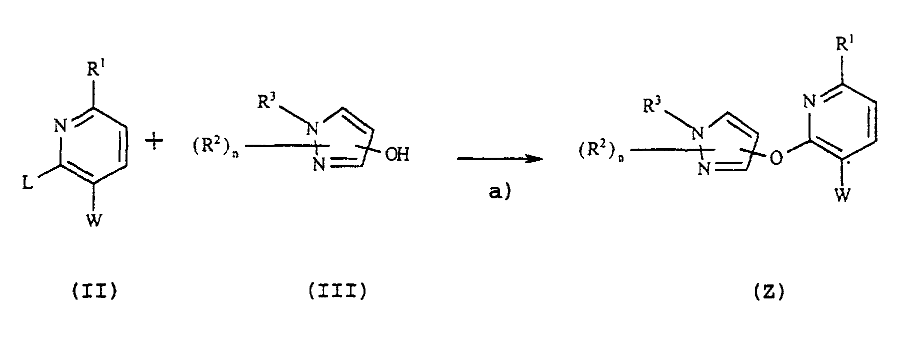

### Hierin bedeutet:

- L: eine für die nucleophile aromatische Substitution übliche Abgangsgruppe wie Fluor, Chlor, Brom oder die Nitrogruppe und insbesondere Chlor oder Fluor;
- W: eine Gruppe, welche den nucleophilen Austausch der Gruppe L ermöglicht oder erleichtert und sich zudem - wie unten beschrieben - in die Gruppe Q der Verbindungen I umwandeln läßt, z.B.: -CO-CO-OR', -CO-CO-NHR', -CO-OR' oder -CO-CH₃ (R' = C₁-C₄-Alkyl), insbesondere -CO-CO-OCH₃.

1a) Diese Umsetzung wird üblicherweise bei Temperaturen von 0 bis 120°C, vorzugsweise 20 bis 60°C durchgeführt.
   Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, sowie Dimethylsulfoxid, Dimethylformamid, Dimethylacetamid, 1,3-Dimethylimidazolidin-2-on und 1,3-Dimethyltetrahydro-2(1H)-pyrimidinon; besonders bevorzugt sind Dimethylsulfoxid, Dimethylacetamid und Dimethylformamid.
   Es können auch Gemische der genannten Lösungsmittel verwendet werden.
   Als Basen kommen allgemein in Betracht: basische, anorganische Verbindungen, z.B. Alkalimetall- und Erdalkalimetallhydride wie Lithiumhydrid, Natriumhydrid, Kaliumhydrid, Calciumhydrid, Alkalimetall- und Erdalkalimetallcarbonate wie Kaliumcarbonat und Calciumcarbonat, ferner Silbercarbonat sowie Alkalimetallhydrogencarbonate wie Natriumhydrogencarbonat, metallorganische Verbindungen sowie Alkalimetall- und Erdalkalimetallalkoholate wie Kaliummethylat, Kalium-tert.butanolat und Dimethoxymagnesium. Außerdem eignen sich organische Basen, z.B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine. Besonders bevorzugt werden Natriumhydrid, Kaliumcarbonat und Kalium-tert.-butanolat.
   Die Basen werden im allgemeinen äquimolar oder gegebenenfalls als Lösungsmittel verwendet.
   Es kann für die Umsetzung vorteilhaft sein, zunächst die Verbindungen III mit Base zu behandeln und das resultierende Salz mit der Verbindung II umzusetzen.
   Es kann für die Umsetzung weiterhin vorteilhaft sein, eine katalytische Menge eines Kronenethers wie z.B. 18-Krone-6 oder 15-Krone-5 oder eines sonstigen üblichen Phasentransferkatalysators zuzusetzen.
   Als Phasentransferkatalysatoren können Ammoniumhalogenide und -tetrafluoroborate wie Benzyltriethylammoniumchlorid, Benzyltributylammoniumbromid, Tetrabutylammoniumchlorid, Hexadecyltrimethylammoniumbromid oder Tetrabutylammoniumtetrafluoroborat sowie Phosphoniumhalogenide wie Tetrabutylphosphoniumchlorid oder Tetraphenylphosphoniumbromid eingesetzt werden.

Die für die Herstellung der Verbindungen I benötigten Ausgangsstoffe II sind in der Literatur bekannt [WO-A 95/06033; J.Heterocycl.Chem. 30, 717 (1993); WO-A 97/17328; WO-A 97/24317] oder können gemäß der zitierten Literatur hergestellt werden.

Beispielsweise können die α-Ketoester IIa (W = -CO-CO-OCH₃) wie in Schema 1 aufgezeigt synthetisiert werden. 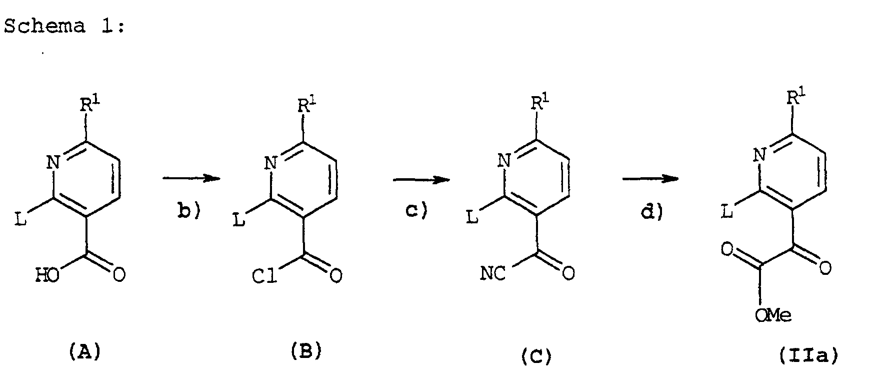
1b) Die Umsetzung der Pyridylcarbonsäure A zum Säurechlorid B erfolgt in an sich bekannter Weise [Houben-Weyl, Ergänzungsband 5, S. 59ff, 225ff und 664ff; J.Heterocycl.Chem, 30, 771 (1993)] mittels üblicher Chlorierungsmittel bei Temperaturen von 0°C bis 150°C, vorzugsweise 10°C bis 100°C ggf. in Gegenwart eines inerten organischen Lösungsmittels. Als Chlorierungsmittel sind alle hierfür üblichen Reagentien geeignet, insbesondere SOCl₂, (COCl)₂, PCl₃, AlCl₃ und PCl₅. Die Chlorierungsmittel werden im allgemeinen im Überschuß oder ggf. als Lösungsmittel verwendet.
1c) Die Umsetzung des Säure-Chlorids B zum Cyanid C erfolgt in an sich bekannter Weise [WO-A 97/29093] bei Temperaturen von 0°C bis 150°C, vorzugsweise 10°C bis 100°C mit einem anorganischen Cyanid in einem inerten organischen Lösungsmittel ggf. im Gemisch mit Wasser in Gegenwart eines üblichen Phasentransferkatalysators (z.B. Tetraalkylammoniumhalogenide wie Tetrabutylammonium-chlorid oder -bromid).
   Als anoganische Cyanide eignen sich Cyanide von Metallen der ersten Hauptgruppe oder der Nebengruppen des Periodensystems, beisielsweise Lithium, Natrium, Kalium, Kupfer und Silber, insbesondere Kupfer und Natrium, sowie organische Cyanide wie Trimethylsilylcyanid.
1d) Die Umsetzung des Cyanids C zum α-Ketoester IIa erfolgt in an sich bekannter Weise im Wege einer Pinner-Reaktion [WO-A 97/29093] bei Temperaturen von 0°C bis 150°C, vorzugsweise 10°C bis 100°C in Gegenwart einer Säure in Methanol als Lösungsmittel.

Die α-Ketoester IIaa, die in 2-Stellung am Pyridinring einen Chlorsubstituenten tragen, sind wie in Schema 2 skizziert in nur einer Stufe ausgehend von 2-Chlor-3-iod- oder 2-Fluor-3-iodpyridinen D in einer Grignard-Reaktion (vgl. Tetrahedron, 52 (1996) 13513-20), bzw. durch ortho-Metallierung von 2-Chlor- oder 2-Fluorpyridinen der Formel E und anschließende Umsetzung mit α-Oxo-α-(1H-imidazol)-essigsäuremethylester (J. Heterocyclic Chem., 34, 789 (1997)) zugänglich.

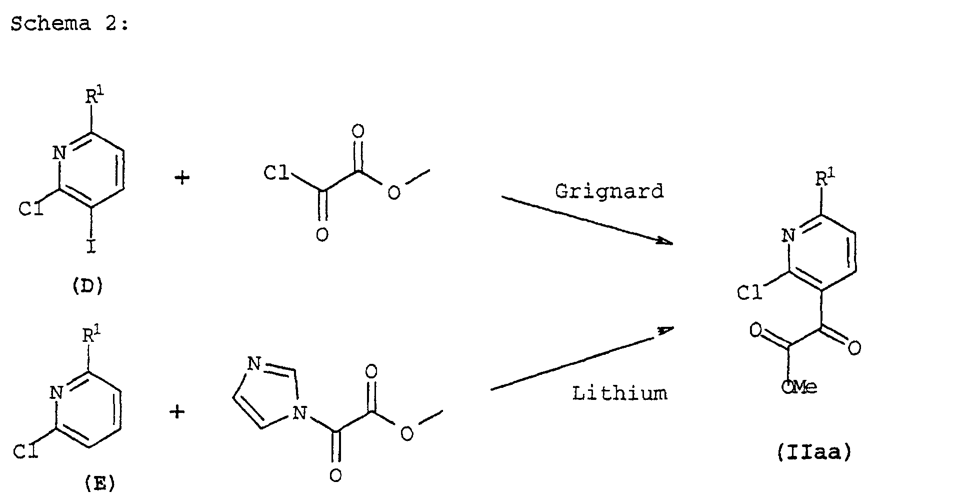

Die Ausgangsstoffe III sind ebenfalls aus der Literatur bekannt oder können nach den dort beschriebenen Verfahren hergestellt werden 3-Hydroxypyrazole: J. Heterocycl. Chem. 30, Seite 49 (1993); Chem. Ber. 107, Seite 1318 (1974); Chem. Pharm. Bull. 19, Seite 1389 (1971); Tetrahedron Lett. 11, Seite 875 (1970); Chem. Heterocycl. Comp. 5, Seite 527 (1969); Chem. Ber. 102, Seite 3260 (1969); Chem. Ber. 109, Seite 261 (1976); J. Org. Chem. 31, Seite 1538 (1966); Tetrahedron 43, Seite 607 (1987); 4-Hydroxypyrazole: CA-A 1 177 081; US-A 4,621,144; JP-A 60/155,160].

2-(Pyrazolyloxy)pyridin-3-ylglyoxylsäuremethylester der Formel Za lassen sich entweder wie eingangs erwähnt durch Umsetzung der α-Ketoester IIa mit 3-Hydroxypyrazolen III gewinnen oder aus geeigneten Vorstufen der Formel Z', in der W für COO-C₁-C₄-Alkyl und COCH₃ steht, darstellen.

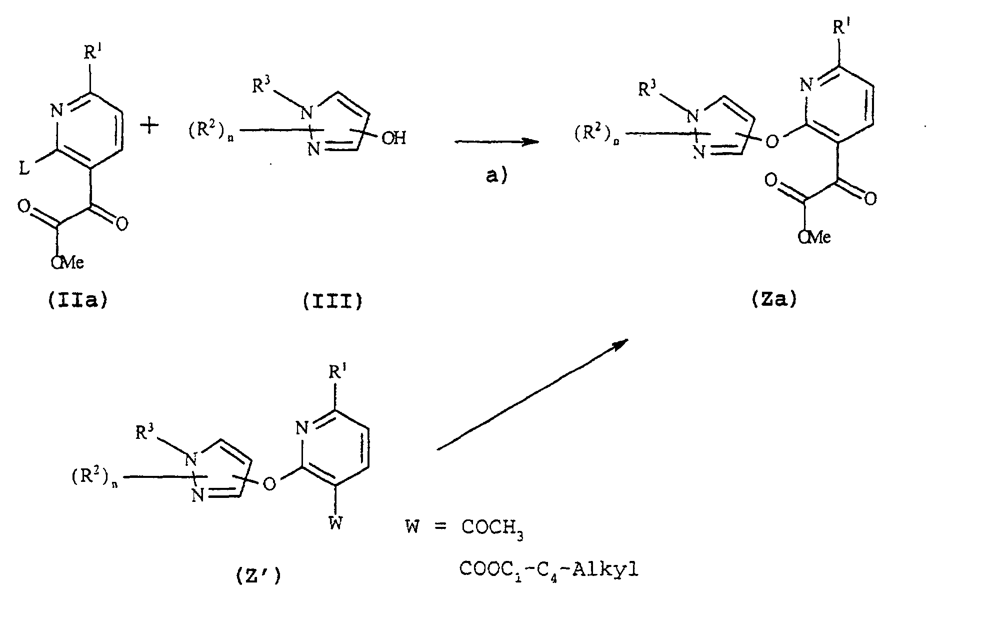

Die folgende Übersicht zeigt, welche an sich bekannten Reaktionen sich für die Umwandlung von Verbindungen Z' in Verbindungen Za eignen:

W = -CO-OR' (R' = C₁-C₄-Alkyl)

Verseifung der -CO-OR'-Gruppe zur Carboxylgruppe (vgl. Verbindungen A in Schema 1);

W = -CO-CH₃

Oxidation der CH₃-Gruppe (vgl.: mit Kaliumpermanganat: Houben-Weyl, Band 4/1b, Seite 594 ff.; mit SOCl₂: Tetrahedron Lett. 1976, Seite 2783 ff.).

Die Verbindungen der Formel Za lassen sich weiter zu den Verbindungen der Formel I umsetzen.

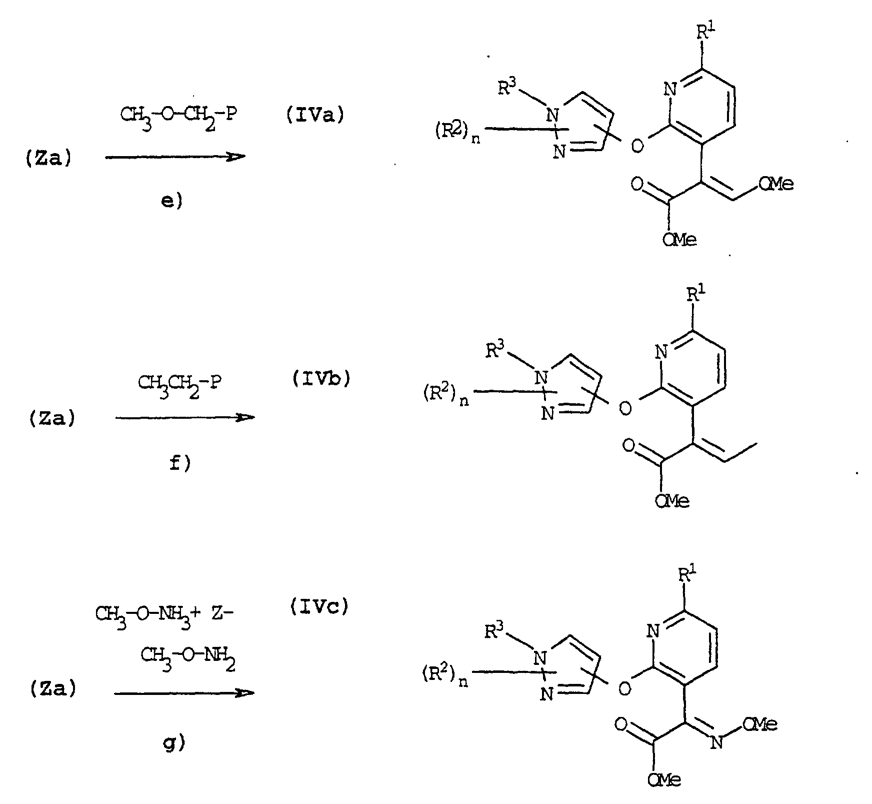

**P** in den Formeln IVa und IVb steht für einen für die Wittig- oder Wittig-Horner Unsetzung geeigneten Phosphonat oder einen Phosphonium-halogenid Rest, insbesondere PO(OCH₃)₂, PO(OCH₂CH₃)₂ und [(C₆H₅)₃P⁺ Cl⁻].

**Z**^{**-**} in der Formel IVc steht für das Anion einer anorganischen Säure, besonders ein Halogenid-anion, insbesondere Chlorid.
1e) Die Umsetzung erfolgt in an sich bekannter Weise [EP-A 513 580; Tetrahedron 3727 (1988); GB-A 2,172,595; WO-A 97/29093 im Wege einer Wittig- oder Wittig-Horner-Reaktion bei Temperaturen von 0°C bis 150°C, vorzugsweise 10°C bis 100°C in einem inerten organischen Lösungsmittel in Gegenwart einer Base.
1f) Die Umsetzung erfolgt in an sich bekannter Weise (vgl. die unter 1e) zitierte Literatur) im Wege einer Wittig- oder Wittig-Horner-Reaktion bei Temperaturen von 0°C bis 150°C, vorzugsweise 10°C bis 100°C in einem inerten organischen Lösungsmittel in Gegenwart einer Base.
1g) Die Umsetzung erfolgt in an sich bekannter Weise (EP-A 493 711) bei Temperaturen von 0°C bis 150°C, vorzugsweise 10°C bis 100°C in einem inerten organischen Lösungsmittel in Gegenwart einer Base mit O-Methylhydroxylamin oder dessen Salz (IVc).
2. Nach einem weiteren Verfahren erhält man die Verbindungen I beispielsweise dadurch, daß man ein Pyridinderivat der Formel VI in an sich bekannter Weise in Gegenwart eines Katalysators, mit einer Verbindung VII in das entsprechende Pyridinderivat der Formel VIII überführt und VIII anschließend in Gegenwart einer Base mit einem Oxim der Formel IV zu I umsetzt.

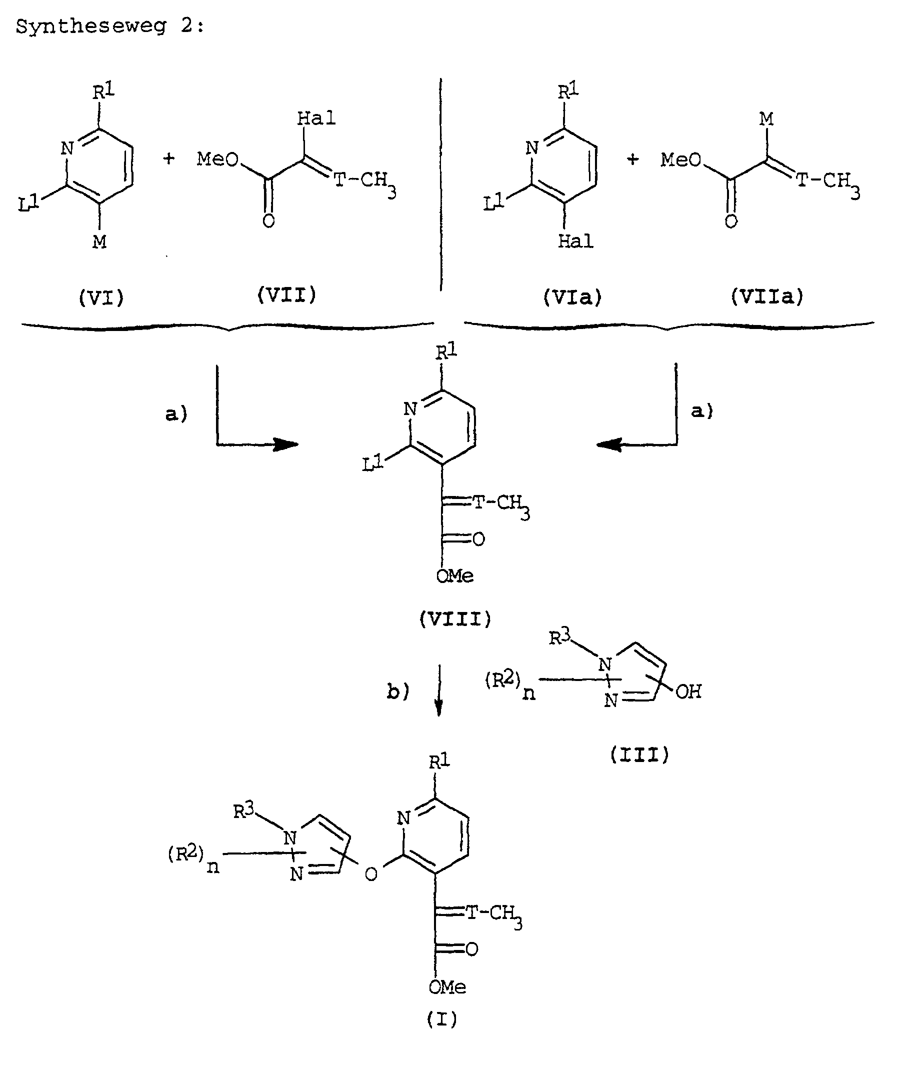

L¹ in den Formeln VI, VIa und VIII steht für eine nucleophil austauschbare Abgangsgruppe wie aliphatische und aromatische Sulfonate und Halogenatome, insbesondere Fluor und Chlor.

M den Formeln VI und VIIa steht für einen metallorganischen Rest, z.B. Tributyl-Zinn(IV), Trimethyl-Zinn(IV), Zink-II-chlorid (ZnCI) oder Bor-II-hydroxid [B(OH)₂], insbesondere Tributyl-Zinn(IV) und Bor-II-hydroxid [B(OH)₂]. Die Verwendung von Trimethyl-Zinn(IV) wird im Hinblick auf dessen hohe Toxizität nur bedingt bevorzugt.

T in den Formeln VII, VIIa, VIII und I steht für CHO oder NO.

Hal in den Formeln VIa und VII steht für ein Halogenatom, insbesondere Brom oder Jod.

In analoger Weise erhält man die Pyridinderivate VIII auch durch Umsetzung eines halogenierten Pyridins der Formel VIa mit einer metallorganischen Verbindung VIIa.
2a) Die Umsetzung der Verbindung VI bzw. VIa mit dem Carbonylderivat VII bzw. VIIa erfolgt in an sich bekannter Weise bei Temperaturen von 0°C bis 150°C, vorzugsweise 10°C bis 100°C in einem inerten organischen Lösungsmittel ggf. in Gegenwart eines Cokatalysators wie CuI. Für den Fall, daß die Umsetzung mit Verbindungen VI bzw. VIIa durchgeführt wird, in denen M für B(OH)₂ steht, erfolgt die Reaktion in Gegenwart von mindestens äquimolaren Mengen einer Base.
   Geeignete Lösungsmittel sind aromatische Kohlenwasserstoffe wie Toluol, o-, m- und p-Xylol, Ether wie Diethylether, Diisopropylether, tert.-Butylmethylether, Dimethoxyethan, Dioxan, Anisol und Tetrahydrofuran, sowie Dimethylformamid und Dimethylacetamid, Wasser und Methanol, besonders bevorzugt N-Methylpyrrolidon. Es können auch Gemische der genannten Lösungsmittel verwendet werden.
   Als Basen für die Kupplung von Verbindungen, in denen M für B(OH)₂ steht, kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetallphosphate wie Kaliumphosphat, Alkalimetall- und Erdalkalimetalloxide wie Lithiumoxid, Natriumoxid, Calziumoxid und Magnesiumoxid, Alkalimetall- und Erdalkalimetallcarbonate wie Lithiumcarbonat und Calziumcarbonat sowie Alkalimetallhydrogencarbonate wie Natriumhydrogencarbonat, sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumcarbonat, Natriumhydrogencarbonat und Lithiumhydroxid.
   Die Basen werden im allgemeinen äquimolar, im Überschuß oder gegebenenfalls als Lösungsmittel verwendet.
   Die Edukte werden im allgemeinen in äquimolaren Mengen miteinander umgesetzt. Es kann für die Ausbeute vorteilhaft sein, VI bzw. VIIa in einem Überschuß bezogen auf VII bzw VIa einzusetzen.
   Die für die Herstellung der Verbindungen I benötigten Ausgangsstoffe sind in der Literatur bekannt [WO-A 95/20569; WO-A 94/24085; Synlett (1) 32-33 (1995); Synlett (4) 356-357 (1996); J.Gen.Chem.USSR 59, 264-272 (1989); Heterocycles 31, 1543-1548 (1990); Tetrahedron 49, 49-64 (1993); J.Chem.Res.Miniprint 11, 4658-4667 (1980)] oder können gemäß der zitierten Literatur hergestellt werden.
2b) Die Umsetzung der Verbindung VIII mit dem Hydroxypyrazol III erfolgt in an sich bekannter Weise bei Temperaturen von 0°C bis 150°C, vorzugsweise 10°C bis 100°C in einem inerten organischen Lösungsmittel in Gegenwart einer Base.
   Geeignete Lösungsmittel sind Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Dimethylsulfoxid, Dimethylformamid und Dimethylacetamid. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallcarbonate wie Lithiumcarbonat und Calziumcarbonat sowie Alkalimetallhydrogencarbonate wie Natriumhydrogencarbonat, sowie Alkalimetall- und Erdalkalimetallalkoholate wie Natriummethanolat, Natriumethanolat, Kaliumethanolat, Kaliumtert.-Butanolat und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kalium-tert.-butylat, Natrium-methylat und Kaliumcarbonat.
   Die Basen werden im allgemeinen äquimolar, im Überschuß oder gegebenenfalls als Lösungsmittel verwendet.
   Die Edukte werden im allgemeinen in äquimolaren Mengen miteinander umgesetzt. Es kann für die Ausbeute vorteilhaft sein, III in einem Überschuß bezogen auf VIII einzusetzen.
2.1 Gemäß den vorstehend beschriebenen Umsetzungsbedingungen erhält man die Verbindungen I auch besonders bevorzugt dadurch, daß man das Pyridin VIa zunächst mit dem Hydroxypyrazol III in das entsprechende Derivat V überführt und V anschließend mit VIIa zu I umsetzt. 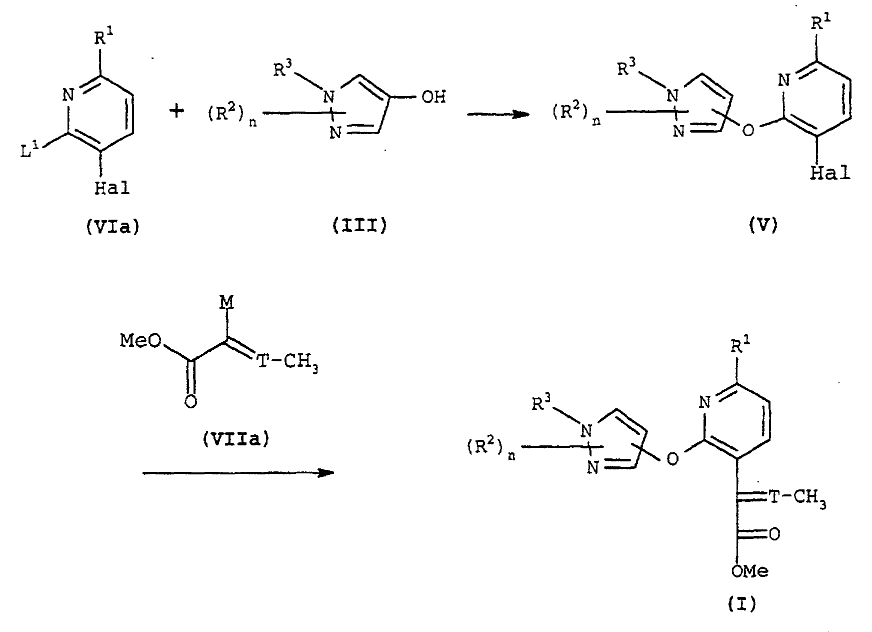
3. Die Verbindungen der Formel I, denen Q für eine Gruppe -C(=NOCH₃)CONH(CH₃) steht, erhält man vorteilhaft dadurch, daß man eine Verbindung der Formel I, in der Q = -C(=NOCH₃)COOCH₃ in an sich bekannter Weise mit Methylamin oder dessen Salz umsetzt.
   Diese Umsetzung erfolgt in an sich bekannter Weise (EP-A 477 631) bei Temperaturen von 0°C bis 150°C, vorzugsweise 10°C bis 100°C in einem inerten organischen Lösungsmittel und gegebenenfalls in Gegenwart einer Base.
   Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, Isopropanol, n-Butanol und tert.-Butanol sowie Dimethylsulfoxid, Dimethylformamid und Dimethylacetamid, besonders bevorzugt Tetrahydrofuran. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallcarbonate wie Lithiumcarbonat und Calziumcarbonat sowie Alkalimetallhydrogencarbonate wie Natriumhydrogencarbonat, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden tertiäre Amine.
   Die Basen werden im allgemeinen äquimolar, im Überschuß oder gegebenenfalls als Lösungsmittel verwendet.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können bei der Herstellung aufgrund ihrer C=Cund C=N-Doppelbindungen in der Gruppe Q als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung der Isomeren nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen, in der behandelten Pflanze oder im zu bekämpfenden Schadpilz oder tierischen Schädling erfolgen.

In Bezug auf die C=N- bzw. C=C-Doppelbindung in der Gruppe Q werden hinsichtlich ihrer Wirksamkeit die E-Isomere der Verbindungen I bevorzugt (Konfiguration bezogen auf die (OCH₃)- bzw. die (CH₃)-Gruppe im Verhältnis zur COOCH₃- bzw. CONHCH₃-Gruppe).

Teil der Erfindung sind auch die Salze der säurebeständigen Verbindungen I, welche basische Zentren, vor allem basische Stickstoffatome enthalten, insbesondere mit Mineralsäuren wie Schwefelsäure und Phosphorsäure oder Lewis-Säuren wie Zinkchlorid. Üblicherweise kommt es hierbei auf die Art des Salzes nicht an. Im Sinne der Erfindung sind solche Salze bevorzugt, die die von Schadpilzen oder tierischen Schädlingen freizuhaltenden Pflanzen, Flächen, Materialien oder Räume nicht schädigen und die Wirkung der Verbindungen I nicht beeinträchtigen. Besonders bedeutsam sind derartige, für landwirtschaftliche Zwecke geeignete Salze.

Die Salze der Verbindungen I sind in an sich bekannter Weise zugänglich, vor allem durch Umsetzen der entsprechenden Verbindungen I mit den genannten Säuren in Wasser oder einem inerten organischen Lösungsmittel bei Temperaturen von -80 bis 120, vorzugsweise 0 bis 60°C.

Bei den eingangs angegebenen Definitionen der Verbindungen I wurden Sammelbegriffe verwendet, die allgemein repräsentativ für die folgenden Gruppen stehen:
Halogen: Fluor, Chlor, Brom und Jod;
C₁-C₄-Alkyl: geradkettige oder verzweigte Alkylgruppen mit 1 bis 4 Kohlenstoffatomen, wie Methyl, Ethyl, Propyl, 1-Methylethyl, Butyl, 1-Methylpropyl, 2-Methylpropyl, 1,1-Dimethylethyl;
C₁-C₄-Halogenalkyl: geradkettige oder verzweigte Alkylgruppen mit 1 bis 4 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
C₁-C₄-Alkoxy sowie der C₁-C₄-Alkoxyteil von C₁-C₄-Alkoxycarbonyl: geradkettige oder verzweigte Alkylgruppen mit 1 bis 4 Kohlenstoffatomen wie vorstehend genannt, welche über ein Sauerstoffatom (-O-) an das Gerüst gebunden sind, z.B. C₁-C₄-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy;
C₁-C₂-Halogenalkoxy: geradkettige Alkylgruppen mit 1 bis 2 Kohlenstoffatomen, wobei in diesen Gruppen teilweise oder vollständig die Wasserstoffatome durch Halogenatome wie vorstehend genannt ersetzt sein können, und wobei diese Gruppen über ein Sauerstoffatom an das Gerüst gebunden sind, z.B. Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome, wie vorstehend genannt, ersetzt sein können.

In Hinblick auf ihre Wirkung bei der Bekämpfung von Schadpilzen und tierischen Schädlingen sind Verbindungen der Formeln Ia und Ib 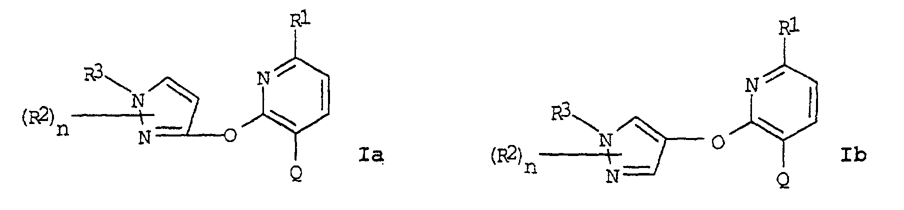 sowie ihre Salze bevorzugt, in denen die Substituenten und der Index n, jeweils für sich allein oder in Kombination, die folgenden Bedeutungen haben:
- n: 0 oder 1;
- R¹: Wasserstoff, Halogen, Halogenmethyl;
- R²: Chlor, Brom, Methyl oder Trifluormethyl:
- R³: Phenyl,Pyridyl und Pyrimidyl, wobei der Phenyl-,Pyridyl- und Pyrimidylrest einen oder, unabhängig voneinander, zwei oder drei der folgenden Substituenten tragen kann: Halogen, Cyano, C₁-C₄-Alkyl, C₁-C₂-Halogenalkyl, C₁-C₄-Alkoxy, C₁-C₂-Halogenalkoxy oder C₁-C₄-Alkoxycarbonyl.
- Q: -C(=CHCH₃) -COOCH₃, -C(=CHOCH₃) -COOCH₃, -C(=NOCH₃)-COOCH₃ oder -C(=NOCH₃)-CONH(CH₃);

Insbesondere bevorzugt sind Verbindungen I sowie ihre Salze, in denen die Substituenten und der Index n die folgenden Bedeutungen haben:
- n: 0 oder 1;
- Q: -C(=CHOCH₃)-COOCH₃,
-C(=NOCH₃)-CONH(CH₃);
- R¹: Wasserstoff, Halogen, Halogenmethyl;
- R²: chlor, Brom, Methyl oder Trifluormethyl:
- R³: Phenyl, wobei der Phenylrest einen oder, unabhängig voneinander, zwei oder drei der folgenden Substituenten tragen kann: Halogen, Cyano, C₁-C₄-Alkyl, C₁-C₂-Halogenalkyl, C₁-C₄-Alkoxy, C₁-C₂-Halogenalkoxy oder C₁-C₄-Alkoxycarbonyl.

Bevorzugt sind auch die Verbindungen der allgemeinen Formel I sowie ihre Salze, in der die Pyrazolyloxygruppe eine der folgenden Bedeutungen hat:

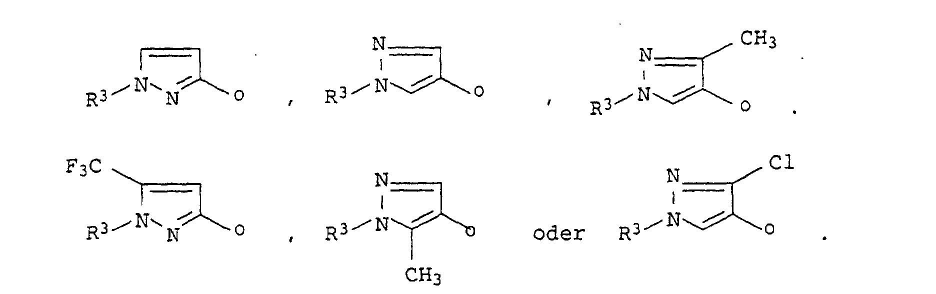

Ganz besonders bevorzugt sind im Hinblick auf ihre biologische Wirkung die in den folgenden Tabellen zusammengestellten Verbindungen, wobei die dem die zentralen Pyridinring benachbarten Doppelbindungen in den unterschiedlichen Gruppen Q stets in der E-Konfiguration vorliegen.

### Tabelle 1

Verbindungen der Formel IA, 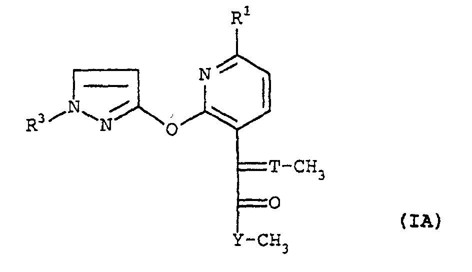 in der T für N-O und Y für O stehen, während die Reste R¹ und R³ die Bedeutungen haben, welche jeweils einer Zeile der Tabelle A entsprechen.

### Tabelle 2

Verbindungen der Formel IA, in der T für N-O und Y für NH stehen, während die Reste R¹ und R³ die Bedeutungen haben, welche jeweils einer Zeile der Tabelle A entsprechen.

### Tabelle 3

Verbindungen der Formel IA, in der T für CH-O und Y für O stehen, während die Reste R¹ und R³ die Bedeutungen haben, welche jeweils einer Zeile der Tabelle A entsprechen.

### Tabelle 4

Verbindungen der Formel IA, in der T für CH und Y für O stehen, während die Reste R¹ und R³ die Bedeutungen haben, welche jeweils einer Zeile der Tabelle A entsprechen.

### Tabelle 5

Verbindungen der Formel IB, 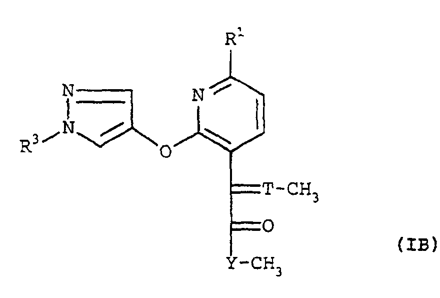 in der T für N-O und Y für O stehen, während die Reste R¹ und R³ die Bedeutungen haben, welche jeweils einer Zeile der Tabelle A entsprechen.

### Tabelle 6

Verbindungen der Formel IB, in der T für N-O und Y für NH stehen, während die Reste R¹ und R³ die Bedeutungen haben, welche jeweils einer Zeile der Tabelle A entsprechen.

### Tabelle 7

Verbindungen der Formel IB, in der T für CH-O und Y für O stehen, während die Reste R¹ und R³ die Bedeutungen haben, welche jeweils einer Zeile der Tabelle A entsprechen.

### Tabelle 8

Verbindungen der Formel IB, in der T für CH und Y für O stehen, während die Reste R¹ und R³ die Bedeutungen haben, welche jeweils einer Zeile der Tabelle A entsprechen.

### Tabelle 9

Verbindungen der Formel IC, 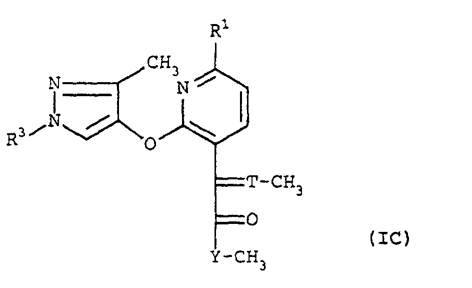 in der T für N-O und Y für O stehen, während die Reste R¹ und R³ die Bedeutungen haben, welche jeweils einer Zeile der Tabelle A entsprechen.

### Tabelle 10

Verbindungen der Formel IC, in der T für N-O und Y für NH stehen, während die Reste R¹ und R³ die Bedeutungen haben, welche jeweils einer Zeile der Tabelle A entsprechen.

### Tabelle 11

Verbindungen der Formel IC, in der T für CH-O und Y für O stehen, während die Reste R¹ und R³ die Bedeutungen haben, welche jeweils einer Zeile der Tabelle A entsprechen.

### Tabelle 12

Verbindungen der Formel IC, in der T für CH und Y für O stehen, während die Reste R¹ und R³ die Bedeutungen haben, welche jeweils einer Zeile der Tabelle A entsprechen.

### Tabelle 13

Verbindungen der Formel ID, 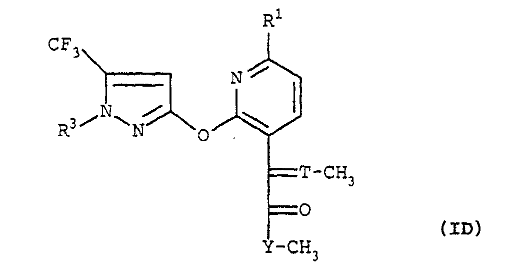 in der T für N-O und Y für O stehen, während die Reste R¹ und R³ die Bedeutungen haben, welche jeweils einer Zeile der Tabelle A entsprechen.

### Tabelle 14

Verbindungen der Formel ID, in der T für N-O und Y für NH stehen, während die Reste R¹ und R³ die Bedeutungen haben, welche jeweils einer Zeile der Tabelle A entsprechen.

### Tabelle 15

Verbindungen der Formel ID, in der T für CH-O und Y für O stehen, während die Reste R¹ und R³ die Bedeutungen haben, welche jeweils einer Zeile der Tabelle A entsprechen.

### Tabelle 16

Verbindungen der Formel ID, in der T für CH und Y für O stehen, während die Reste R¹ und R³ die Bedeutungen haben, welche jeweils einer Zeile der Tabelle A entsprechen.

### Tabelle 17

Verbindungen der Formel IE, 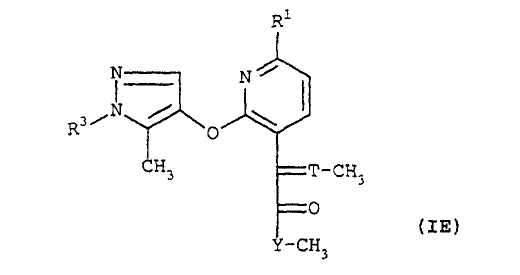 in der T für N-O und Y für O stehen, während die Reste R¹ und R³ die Bedeutungen haben, welche jeweils einer Zeile der Tabelle A entsprechen.

### Tabelle 18

Verbindungen der Formel IE, in der T für N-O und Y für NH stehen, während die Reste R¹ und R³ die Bedeutungen haben, welche jeweils einer Zeile der Tabelle A entsprechen.

### Tabelle 19

Verbindungen der Formel IE, in der T für CH-O und Y für O stehen, während die Reste R¹ und R³ die Bedeutungen haben, welche jeweils einer Zeile der Tabelle A entsprechen.

### Tabelle 20

Verbindungen der Formel IE, in der T für CH und Y für O stehen, während die Reste R¹ und R³ die Bedeutungen haben, welche jeweils einer Zeile der Tabelle A entsprechen.

### Tabelle 21

Verbindungen der Formel IF, 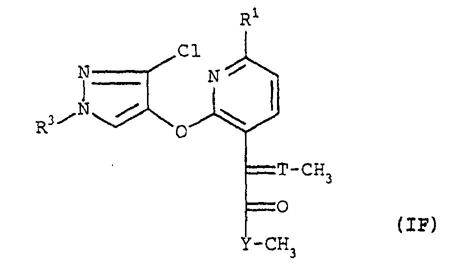 in der T für N-O und Y für O stehen, während die Reste R¹ und R³ die Bedeutungen haben, welche jeweils einer Zeile der Tabelle A entsprechen.

### Tabelle 22

Verbindungen der Formel IF, in der T für N-O und Y für NH stehen, während die Reste R¹ und R³ die Bedeutungen haben, welche jeweils einer Zeile der Tabelle A entsprechen.

### Tabelle 23

Verbindungen der Formel IF, in der T für CH-O und Y für O stehen, während die Reste R¹ und R³ die Bedeutungen haben, welche jeweils einer Zeile der Tabelle A entsprechen.

### Tabelle 24

Verbindungen der Formel IF, in der T für CH und Y für O stehen, während die Reste R¹ und R³ die Bedeutungen haben, welche jeweils einer Zeile der Tabelle A entsprechen.

**Tabelle A**

| **Nr**. | **R**^{**1**} | **R3** |
|---|---|---|
| A.1 | H | C₆H₅ |
| A.2 | Cl | C₆H₅ |
| A.3 | Br | C₆H₅ |
| A.4 | F | C₆H₅ |
| A.5 | CCl₃ | C₆H₅ |
| A.6 | CF₃ | C₆H₅ |
| A.7 | H | 2-F-C₆H₄ |
| A.8 | H | 3-F-C₆H₄ |
| A.9 | H | 4-F-C₆H₄ |
| A.10 | Cl | 4-F-C₆H₄ |
| A.11 | Br | 4-F-C₆H₄ |
| A.12 | F | 4-F-C₆H₄ |
| A.13 | CCl₃ | 4-F-C₆H₄ |
| A.14 | CF₃ | 4-F-C₆H₄ |
| A.15 | H | 2,3-F₂-C₆H₃ |
| A.16 | H | 2,4-F₂-C₆H₃ |
| A.17 | Cl | 2,4-F₂-C₆H₃ |
| A.18 | Br | 2,4-F₂-C₆H₃ |
| A.19 | F | 2,4-F₂-C₆H₃ |
| A.20 | CCl₃ | 2,4-F₂-C₆H₃ |
| A.21 | CF₃ | 2,4-F₂-C₆H₃ |
| A.22 | H | 2,5-F₂-C₆H₃ |
| A.23 | H | 2,6-F₂-C₆H₃ |
| A.24 | H | 3,4-F₂-C₆H₃ |
| A.25 | H | 3,5-F₂-C₆H₃ |
| A.26 | Cl | 3,5-F₂-C₆H₃ |
| A.27 | Br | 3,5-F₂-C₆H₃ |
| A.28 | F | 3,5-F₂-C₆H₃ |
| A.29 | CCl₃ | 3,5-F₂-C₆H₃ |
| A.30 | CF₃ | 3,5-F₂-C₆H₃ |
| A.31 | H | 2-Cl-C₆H₄ |
| A.32 | H | 3-Cl-C₆H₄ |
| A.33 | H | 4-Cl-C₆H₄ |
| A.34 | Cl | 4-Cl-C₆H₄ |
| A.35 | Br | 4-Cl-C₆H₄ |
| A.36 | F | 4-Cl-C₆H₄ |
| A.37 | CCl₃ | 4-Cl-C₆H₄ |
| A.38 | CF₃ | 4-Cl-C₆H₄ |
| A.39 | H | 2,3-Cl₂-C₆H₃ |
| A.40 | H | 2,4-Cl₂-C₆H₃ |
| A.41 | Cl | 2,4-Cl₂-C₆H₃ |
| A.42 | Br | 2,4-Cl₂-C₆H₃ |
| A.43 | F | 2,4-Cl₂-C₆H₃ |
| A.44 | CCl₃ | 2,4-Cl₂-C₆H₃ |
| A.45 | CF₃ | 2,4-Cl₂-C₆H₃ |
| A.46 | H | 2,5-Cl₂-C₆H₃ |
| A.47 | H | 2,6-Cl₂-C₆H₃ |
| A.48 | H | 3,4-Cl₂-C₆H₃ |
| A.49 | H | 3,5-Cl₂-C₆H₃ |
| A.50 | Cl | 3,5-Cl₂-C₆H₃ |
| A.51 | Br | 3,5-Cl₂-C₆H₃ |
| A.52 | F | 3,5-Cl₂-C₆H₃ |
| A.53 | CCl₃ | 3,5-Cl₂-C₆H₃ |
| A.54 | CF₃ | 3,5-Cl₂-C₆H₃ |
| A.55 | H | 2,3,4-Cl₃-C₆H₂ |
| A.56 | H | 2,3,5-Cl₃-C₆H₂ |
| A.57 | H | 2,3,6-Cl₃-C₆H₂ |
| A.58 | H | 2,4,5-Cl₃-C₆H₂ |
| A.59 | H | 2,4,6-Cl₃-C₆H₂ |
| A.60 | H | 3,4,5-Cl₃-C₆H₂ |
| A.61 | H | 2-Br-C₆H₄ |
| A.62 | H | 3-Br-C₆H₄ |
| A.63 | H | 4-Br-C₆H₄ |
| A.64 | H | 2,4-Br₂-C₆H₃ |
| A.65 | H | 2-Br, 4-F-C₆H₃ |
| A.66 | H | 2-Br, 4-Cl-C₆H₃ |
| A.67 | H | 2-F, 4-Cl-C₆H₃ |
| A.68 | H | 3-F, 4-Cl-C₆H₃ |
| A.69 | H | 3-Cl, 5-F-C₆H₃ |
| A.70 | H | 2-Cl, 4-F-C₆H₃ |
| A.71 | H | 2-CN-C₆H₄ |
| A.72 | H | 3-CN-C₆H₄ |
| A.73 | H | 4-CN-C₆H₄ |
| A.74 | H | 3-CN, 4-Cl-C₆H₃ |
| A.75 | H | 2-CH₃-C₆H₄ |
| A.76 | H | 3-CH₃-C₆H₄ |
| A.77 | H | 4-CH₃-C₆H₄ |
| A.78 | Cl | 4-CH₃-C₆H₄ |
| A.79 | Br | 4-CH₃-C₆H₄ |
| A.80 | F | 4-CH₃-C₆H₄ |
| A.81 | CCl₃ | 4-CH₃-C₆H₄ |
| A.82 | CF₃ | 4-CH₃-C₆H₄ |
| A.83 | H | 2,4-(CH₃)₂-C₆H₃ |
| A.84 | Cl | 2,4-(CH₃)₂-C₆H₃ |
| A.85 | Br | 2,4-(CH₃)₂-C₆H₃ |
| A.86 | F | 2,4-(CH₃)₂-C₆H₃ |
| A.87 | CCl₃ | 2,4-(CH₃)₂-C₆H₃ |
| A.88 | CF₃ | 2,4-(CH₃)₂-C₆H₃ |
| A.89 | H | 2,5-(CH₃)₂-C₆H₃ |
| A.90 | H | 2,5-(CH₃)₂-C₆H₃ |
| A.91 | H | 2,6-(CH₃)₂-C₆H₃ |
| A.92 | H | 3,4-(CH₃)₂-C₆H₃ |
| A.93 | H | 3,5-(CH₃)₂-C₆H₃ |
| A.94 | H | 2,4,6-(CH₃)₃-C₆H₂ |
| A.95 | H | 3,4,5-(CH₃)₃-C₆H₂ |
| A.96 | H | 2-CH₃, 4-Cl-C₆H₃ |
| A.97 | H | 2-Cl, 4-CH₃-C₆H₃ |
| A.98 | H | 3-CH₃, 4-Cl-C₆H₃ |
| A.99 | H | 3-Cl, 5-CH₃-C₆H₃ |
| A.100 | H | 2-CN, 4-CH₃-C₆H₃ |
| A.101 | H | 2-CH₃, 4-CN-C₆H₃ |
| A.102 | H | 4- (C₂H₅) -C₆H₄ |
| A.103 | H | 4-[C(CH₃)₃]-C₆H₄ |
| A.104 | H | 2-CF₃-C₆H₄ |
| A.105 | H | 3-CF₃-C₆H₄ |
| A.106 | H | 4-CF₃-C₆H₄ |
| A.107 | H | 3,5-(CF₃)₂-C₆H₃ |
| A.108 | H | 2-Cl, 4-CF₃-C₆H₃ |
| A.109 | H | 2-OCH₃-C₆H₄ |
| A.110 | H | 3-OCH₃-C₆H₄ |
| A.111 | H | 4-OCH₃-C₆H₄ |
| A.112 | H | 2,4-(OCH₃)₂-C₆H₃ |
| A.113 | H | 3,4- (OCH₃)₂-C₆H₃ |
| A.114 | H | 2,5-(OCH₃)₂-C₆H₃ |
| A.115 | H | 3,5-(OCH₃)₂-C₆H₃ |
| A.116 | H | 3,4,5-(OCH₃)₃-C₆H₂ |
| A.117 | H | 2-CH₃, 4-OCH₃-C₆H₃ |
| A.118 | H | 2-Cl, 4-OCH₃-C₆H₃ |
| A.119 | H | 4-OCF₃-C₆H₄ |
| A.120 | H | 2-OCHF₂-C₆H₄ |
| A.121 | H | 3-OCHF₂-C₆H₄ |
| A.122 | H | 4-OCHF₂-C₆H₄ |
| A.123 | H | 4-(OCF₂CHF₂)-C₆H₄ |
| A.124 | H | 2-F, 4-OCHF₂-C₆H₃ |
| A.125 | H | 4-(OCH₂CH₃)-C₆H₄ |
| A.126 | H | 4-[OC(CH₃)₃]-C₆H₄ |
| A.127 | H | 3-(CO₂CH₃)-C₆H₄ |
| A.128 | H | 4-(CO₂CH₃)-C₆H₄ |
| A.129 | H | 4-[CO₂C(CH₃)₃]-C₆H₄ |
| A.130 | H | Pyridin-2-yl |
| A.131 | H | 5-Cl-Pyridin-2-yl |
| A.132 | H | 3,5-Cl₂-Pyridin-2-yl |
| A.133 | H | 5-CF₃-Pyridin-2-yl |
| A.134 | H | 3-Cl, 5-CF₃-Pyridin-2-yl |
| A.135 | H | 3-Cl-Pyridin-2-yl |
| A.136 | H | 4-Cl-Pyrimidin-6-yl |
| A.137 | H | 6-CF₃-Pyrimidin-4-yl |

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten*, *Deuteromyceten*, *Phycomyceten* und *Basidiomyceten*, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen und Erdnüssen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen der Formel I sind außerdem geeignet, tierische Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor zur Bekämpfung tierischer Schädlinge eingesetzt werden. Insbesondere eignen sie sich zur Bekämpfung der folgenden tierischen Schädlinge:
- Insekten aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Helioverpa armigera, Helioverpa virescens, Helioverpa zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis,
- Käfer (Coleoptera), z.B. Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Otiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria,
- Zweiflügler (Diptera), z.B. Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea und Tipula paludosa,
- Thripse (Thysanoptera), z.B. Frankliniella fusca, Frankliniella occidentalis, Frankliniella tritici, Scirtothrips citri, Thrips oryzae, Thrips palmi und Thrips tabaci,
- Hautflügler (Hymenoptera), z.B. Athalia rosae, Atta cephalotes, Atta sexdens, Atta texana, Hoplocampa minuta, Hoplocampa testudinea, Monomorium pharaonis, Solenopsis geminata und Solenopsis invicta,
- Wanzen (Heteroptera), z.B. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor,
- Pflanzensauger (Homoptera), z.B. Acyrthosiphon onobrychis, Adelges laricis, Aphidula nasturtii, Aphis fabae, Aphis gossypii, Aphis pomi, Aphis sambuci, Brachycaudus cardui, Brevicoryne brassicae, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphum rosae, Megoura viciae, Metopolophium dirhodum, Myzus persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum und Viteus vitifolii,
- Termiten (Isoptera), z.B. Calotermes flavicollis, Leucotermes flavipes, Reticulitermes lucifugus und Termes natalensis,
- Geradflügler (Orthoptera), z.B. Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus,
- Arachnoidea wie Spinnentiere (Acarina), z.B. Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius und Tetranychus urticae,
- Nematoden wie Wurzelgallennematoden, z.B. Meloidogyne hapla, Meloidogyne incognita, Meloidogyne javanica, Zysten-bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus und Pratylenchus goodeyi.

Die Aufwandmenge an Wirkstoff zur Bekämpfung von tierischen Schädlingen beträgt unter Freilandbedingungen 0,1 bis 2,0, vorzugsweise 0,2 bis 1,0 kg/ha.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält maneine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid;
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan;
- Amine wie 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, (8-(1,1-Dimethylethyl)-N-ethyl-N-propyl-1,4-dioxaspiro[4.5]decan-2-methanamin;
- Azole wie 1-[2-(2,4-Dichlorphenyl)-4-ethyl-1,3-dioxolan-2-ylethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, 1-[2-(2,4-Dichlorphenyl)-pentyl]-1H-1,2,4-triazol, 2,4'-Difluor-a-(1H-1,2,4-triazolyl-1-methyl)-benzhydrylalkohol, 1-((bis-(4-Fluorphenyl)-methylsilyl)-methyl)-1H-1,2,4-triazol, 1-[2RS,4RS;-2RS,4SR)-4-brom-2-(2,4-dichlorphenyl)tetrahydrofuryl]-1H-1,2,4-triazol, 2--(4-Chlorphenyl)-3-cyclopropyl-1-(1H-1,2,4-triazol-1-yl)-butan-2-ol, (+)-4-Chlor-4-[4-methyl-2-(1H-1,2,4-triazol-1-ylmethyl)-1,3-dioxolan-2-yl]-phenyl-4-chlorphenylether, (E)-(R,S)-1-(2,4-dichlorphenyl)-4,4-dimethyl-2-(1H-1,2,4-triazol-1-yl)pent-1-en-3-ol, 4-(4-Chlorphenyl)-2-phenyl-2-(1H-1,2,4,-triazolylmethyl)-butyronitril, 3-(2,4-dichlorphenyl)-6-fluor-2-(1H-1,2,4-triazol-1-yl)chinazolin-4(3H)-on, (R,S)-2-(2,4-Dichlorphenyl)-1-H-1,2,4-triazol-1-yl)-hexano-2-ol, (1RS,5RS;1RS,5SR)-5-(4-chlorbenzyl)-2,2-dimethyl-1-(1H-1,2,4-triazol-1-ylmethyl)cyclopentanol, (R,S)-1-(4-chlorphenyl)-4,4-dimethyl-3-(1H-1,2,4-triazol-1-ylmethyl)pentan-3-ol, (+)-2-(2,4,-Dichlorphenyl)-3-(1H-1,2,4-triazolyl)-propyl-1,1,2,2-tetrafluorethylether, (E)-1-[1-[4-Chlor-2-trifluormethyl)-phenyl]-imino)-2-propoxyethyl]-1H-imidazol, 2-(4-Chlorphenyl)-2-(1H-1,2,4-triazol-1-ylmethyl)-hexannitril;
- α-(2-Chlorphenyl)-α-(4-chlorphenyl)-5-pyrimidin-methanol, 5-Butyl-2-dimethylamino-4-hydroxy-6-methyl-pyrimidin, Bis- (p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol;
- Strobilurine wie Methyl-E-methoxyimino-[α-(o-tolyloxy)-o-tolyl]acetat, Methyl-E-2-{2-[6-(2-cyanophenoxy}-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid;
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl] -anilin;
- Phenylpyrrole wie 4-(2,2-Difluor-1,3-benzodioxol-4-yl)-pyrrol-3-carbonitril;
- Zimtsäureamide wie 3-(4-Chlorphenyl)-3-(3,4-dimethoxyphenyl)-acrylsäuremorpholid;
- sowie verschiedene Fungizide, wie Dodecylguanidinacetat, 3-[3-(3,5-Dimethyl-2-oxycyclohexyl)-2-hydroxyethyl]-glutarimid, N-Methyl-, N-ethyl-(4-trifluormethyl,-2-[3',4'-dimethoxyphenyl]-benzoesäureamid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, N-(3-Chlor-2,6-dinitro-4-trifluormethyl-phenyl)-5-trifluormethyl-3-chlor-2-aminopyridin.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle B mit physikalischen Angaben aufgeführt.
1. Herstellung von Methoxyimino-[2-(1-(4-chlorphenyl)-3-pyrazolyloxy)-pyridin-3-yl]-essigsäuremethylester (Verbindung I.1 der Tabelle B) 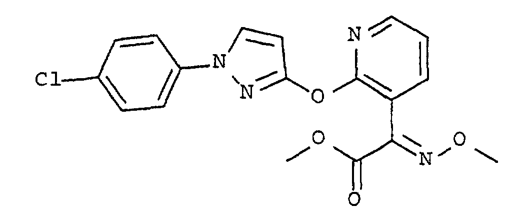
   1a. [2-(1-(4-Chlorphenyl)-3-pyrazolyloxy)-pyridin-3-yl]-glyoxylsäuremethylester 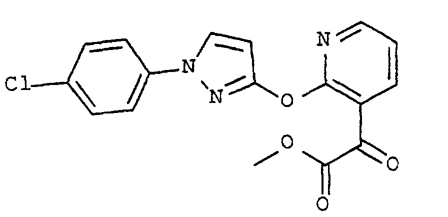
      Zu einer Lösung von 7.4g (38.1 mmol) 1-(4-Chlorphenyl)-3-hydroxypyrazol in 15 mL tert.-Butanol wurde bei 60°C portionsweise 4.7g (42 mmol) Kalium-tert.-butylat zugegeben. Nach einstündigem Rühren bei selbiger Temperatur wurde das Lösungsmittel bei vermindertem Druck entfernt. Der Rückstand wurde in 25 mL abs. Dimethylsulfoxid gelöst, anschließend wurde eine Lösung von 8.0g (38.1 mmol) (2-Chlorpyridin-3-yl) -glyoxylsäuremethylester in 10 mL abs. Dimethylsulfoxid so schnell zugetropft, daß die Temperatur 30°C nicht überstieg. Nach 30 Minuten bei Raumtemperatur wurde 50 mL 0.5 N HCl zugegeben und das Reaktionsgemisch mehrfach mit Essigsäureethylester extrahiert. Die vereinten organischen Phasen wurden mit Wasser gewaschen, über Natriumsulfat getrocknet. Schließlich wurde das Lösungsmittel bei vermindertem Druck entfernt.
      Der so erhaltene Rückstand wurde säulenchromatographisch über Kieselgel mit Cyclohexan/Essigsäureethylester (10:1) als Elutionsmittel gereinigt. Man erhielt 5.5g (40%) der Titelverbindung als helles Öl.
   1.b Methoxyimino-[2-(1-(4-chlorphenyl)-3-pyrazolyloxy)-pyridin-3-yl]-essigsäuremethylester
      Eine Mischung aus 2.7g (7.3 mmol) [2-(1-(4-chlorphenyl)-3-pyrazolyloxy)-pyridin-3-yl]-glyoxylsäuremethylester (Beispiel 1a.), 1.16g Pyridin und 25 mL Methanol wurde mit 0.67 g (8.0 mmol) O-Methylhydroxylamin-Hydrochlorid versetzt und 1.5 Stunden bei Raumtemperatur gerührt. Der entstandene Niederschlag wurde abgetrennt und mit eiskaltem Methanol gewaschen. Der Rückstand wurde bei vermindertem Druck vom Lösungsmittel befreit. Man erhielt 1.2 g (42%) der Titelverbindung als weißen Feststoff.
2. Methoxyimino-[2-(1-(4-chlorphenyl)-3-pyrazolyloxy))-pyridin-3-yl]-essigsäuremethylamid (Verbindung I.2 der Tabelle B) 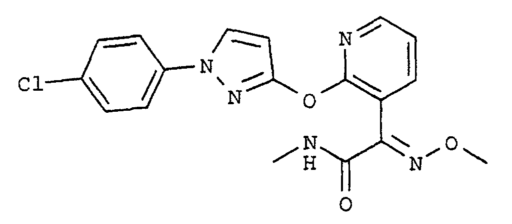
   Eine Mischung aus 0.65g (1.7 mmol) Methoxyimino-[2-(1-(4-chlorphenyl)-3-pyrazolyloxy))-pyridin-3-yl]-essigsäuremethyl -ester (Beispiel 1.) und 3.2g 40%iger wässriger Methylaminlösung in 2 mL Tetrahydrofuran wurden 0.5 Stunden unter Rückfluß erhitzt. Die Reaktionsmischung wurde bei vermindertem Druck vom Lösungsmittel befreit. Der Rückstand wurde mit Pentan/Wasser verrührt, der Feststoff abgetrennt, mit Wasser gewaschen und unter vermindertem Druck getrocknet. Man erhielt 0.55g (84%) der Titelverbindung als weißen Feststoff.
3. MethOxy-[2-(1-(4-chlorphenyl)-3-pyrazolyloxy))-pyridin-3-yl]-acrylsäuremethylester (Verb. I.3 der Tabelle B) 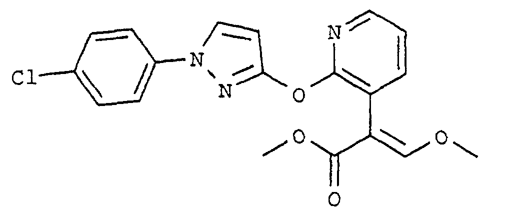
   Eine Mischung aus 1.86g (5.4 mmol) Methoxymethyl-triphenylphosphoniumchlorid und 0.98g (5.4 mmol) Natriummethylatlösung (30%ig in Methanol) in 15 mL Dimethylformamid wurden 10 Minuten bei Raumtemperatur gerührt. Im Anschluß daran wurde die Reaktionsmischung mit 1.0g (2.72 mmol) [2-(1-(4-Chlorphenyl)-3-pyrazolyloxy)-pyridin-3-yl]-glyoxylsäuremethylester (Beispiel 1a.), gelöst in 5 mL Dimethylformamid, versetzt. Die Reaktionsmischung wurde 2 Stunden bei Raumtemperatur gerührt, mit 20 mL Wasser hydrolysiert. Anschließend wurde die wässrige Phase mit Methyl-tert.butylether extrahiert. Die vereinten organischen Phasen wurden über Natriumsulfat getrocknet und schließlich wurde das Lösungsmittel bei vermindertem Druck entfernt. Man erhielt 0.6g (57%) der Titelverbindung in Form eines gelben Feststoffes.

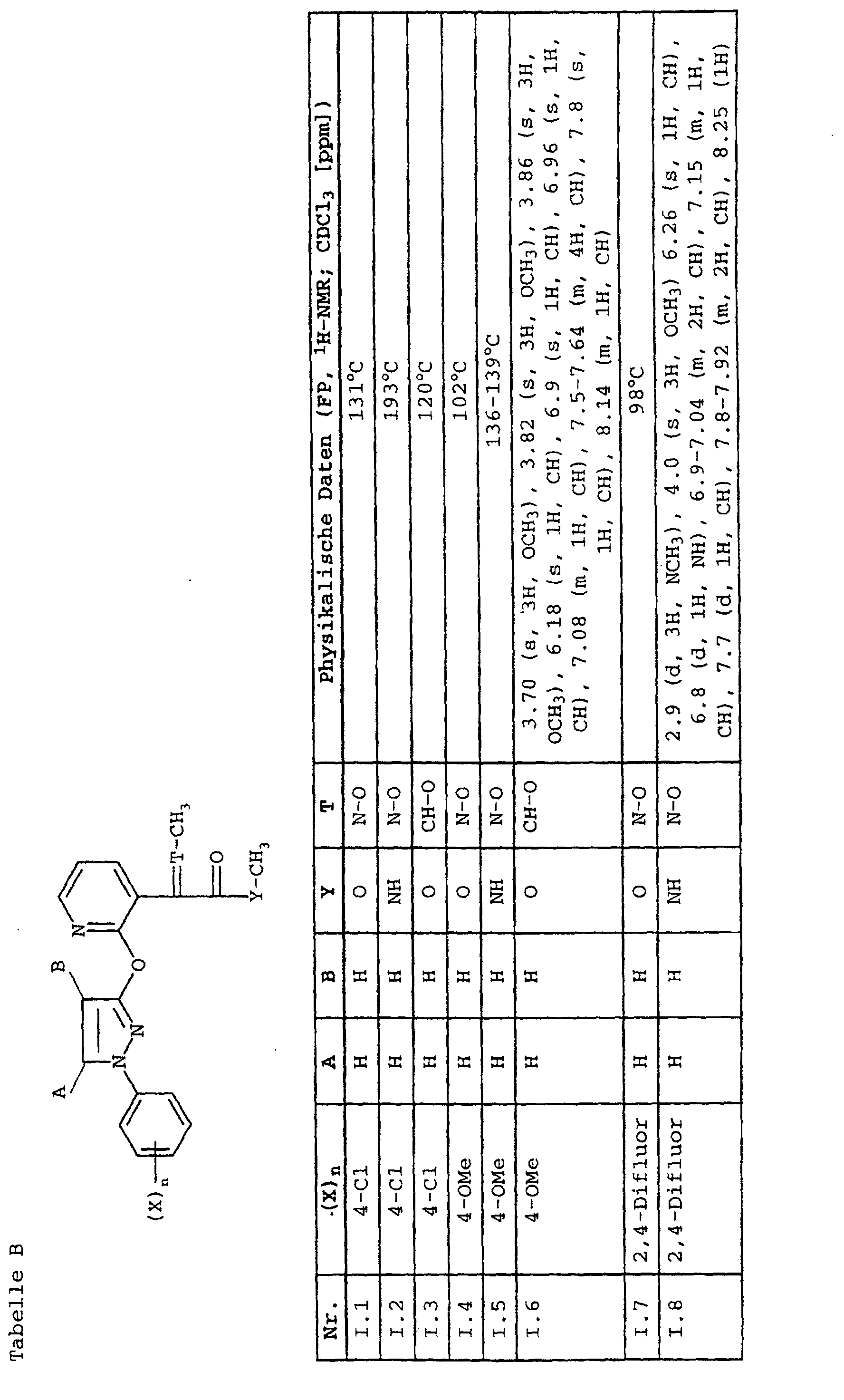

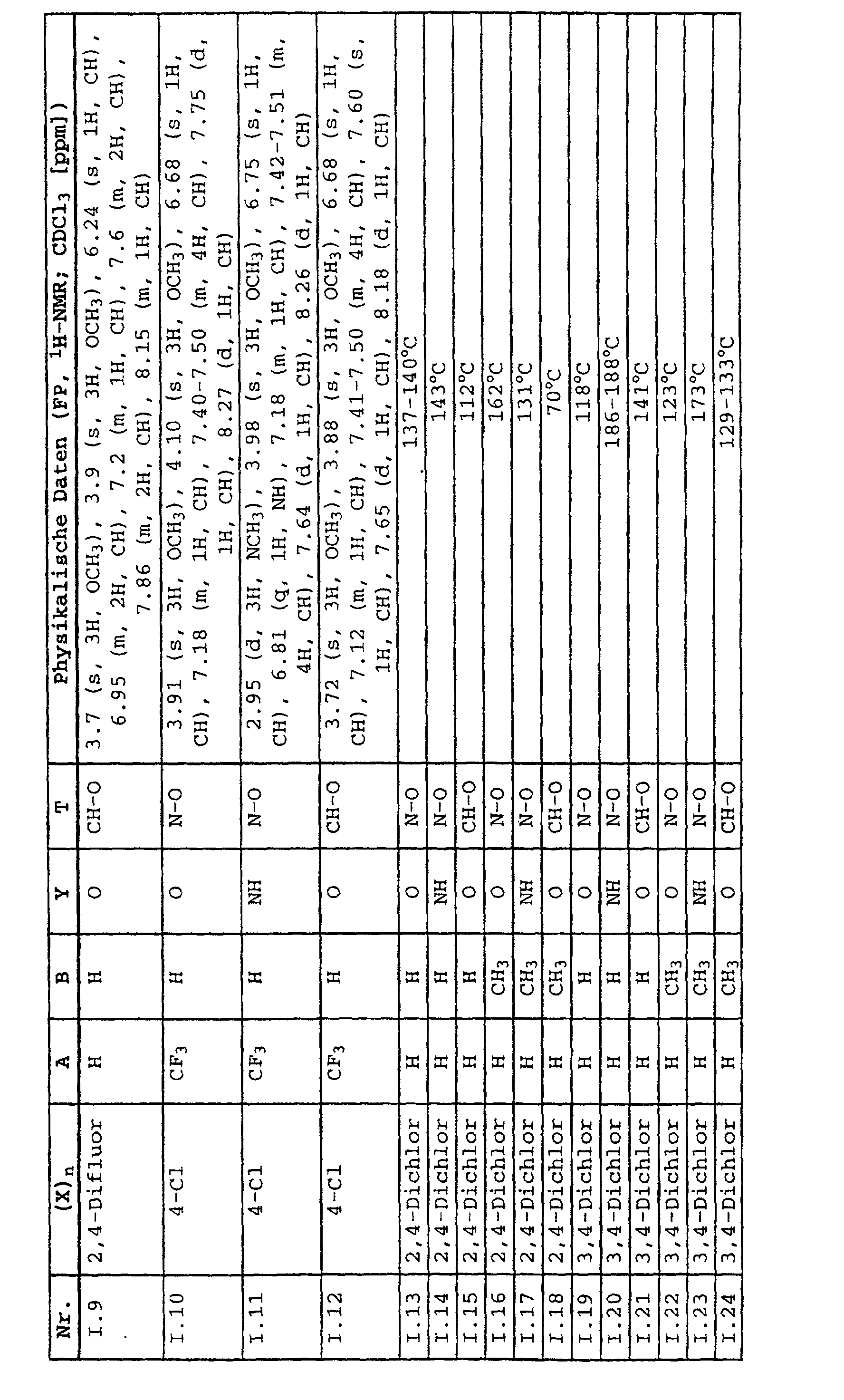

### Anwendungsbeispiele

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

Als Vergleichsverbindung dienten die aus WO-A 97/24332 bekannten Verbindungen A-C

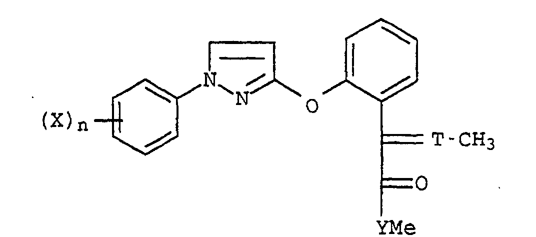

| Nr. | Tabelle D WO-A 97/24332 | (X)ₙ | Y | T |
|---|---|---|---|---|
| A | D.003 | 4-Cl | O | CH-O |
| B | D.008 | 2,4-Dichlor | NH | N-O |
| C | D.007 | 2,4-Dichlor | O | N-O |

### 1) Protektive Wirkung gegen Erysiphe graminis var. tritici (Weizenmeh1tau)

Blätter von in Töpfen gewachsenen Weizenkeimlingen der Sorte "Frühgold" wurden mit wäßriger Wirkstoffaufbereitung, die aus einer Stammlösung bestehend aus 10 % Wirkstoff, 63 % Cyclohexanon und 27 % Emulgiermittel angesetzt wurde, bis zur Tropfnäße besprüht und 24 Stunden nach dem Antrocknen des Spritzbelages mit Sporen des Weizenmehltaus (*Erysiphe graminis* forma specialis *tritici*) bestäubt. Die Versuchspflanzen wurden anschließend im Gewächshaus bei Temperaturen zwischen 20 und 22° C und 75 bis 80 % relativer Luftfeuchtigkeit aufgestellt. Nach 7 Tagen wurde das Ausmaß der Mehltauentwicklung visuell in % Befall der gesamten Blattfläche ermittelt.

In diesem Test zeigte die mit 16 ppm der erfindungsgemäßen Verbindung 1.3 behandelten Pflanzen einen Befall von 5%, während die mit 16 ppm der Vergleichsverbindung A behandelten Pflanzen zu 60% und die unbehandelten Pflanzen (Kontrolle) zu 80% befallen waren.

### 2) Dauer-Wirkung gegen Plasmopara viticola

Blätter von Topfreben der Sorte "Müller-Thurgau" wurden mit wäßriger Wirkstoffauf-bereitung, die mit einer Stammlösung aus 10 % Wirkstoff, 63 % Cyclohexanon und 27 % Emulgiermittel angesetzt wurde, bis zur Tropfnäße besprüht. Um die Dauerwirkung der Substanzen beurteilen zu können, wurden die Pflanzen nach dem Antrocknen des Spritzbelages für 7 Tage im Gewächshaus aufgestellt. Erst dann wurden die Blätter mit einer wäßrigen Zoosporenaufschwemmung von *Plasmopara viticola* inokuliert. Danach wurden die Reben zunächst für 48 Stunden in einer wasserdampfgesättigten Kammer bei 24° C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

In diesem Test zeigte die mit 16 ppm der erfindungsgemäßen Verbindung I.3 behandelten Pflanzen keinen Befall, während die mit 16 ppm der Vergleichsverbindung A behandelten Pflanzen zu 40% und die unbehandelten Pflanzen (Kontrolle) zu 75% befallen waren.

### 3) Protektive Wirksamkeit gegen Pyricularia oryzae

Blätter von in Töpfen gewachsenen Reiskeimlingen der Sorte "Tai-Nong 67" wurden mit wäßriger Wirkstoffaufbereitung, die mit einer Stammlösung aus 10 % Wirkstoff, 63 % Cyclohexanon und 27 % Emulgiermittel angesetzt wurde, bis zur Tropfnäße besprüht. Am folgenden Tag wurden die Pflanzen mit einer wäßrigen Sporensuspension von *Pyricularia oryzae* inokuliert. Anschließend wurden die Versuchspflanzen in Klimakammern bei 22 - 24° C und 95 - 99 % relativer Luftfeuchtigkeit für 6 Tage aufgestellt. Dann wurde das Ausmaß der Befallsentwicklung auf den Blättern visuell ermittelt.

In diesem Test zeigte die mit 16 ppm der erfindungsgemäßen Verbindung I.3 behandelten Pflanzen einen Befall von 15%, während die mit 16 ppm der Vergleichsverbindung A behandelten Pflanzen zu 60% und die unbehandelten Pflanzen (Kontrolle) zu 85% befallen waren.

### 4) - Wirksamkeit gegen Botrykis cinerea an Paprikablättern

Paprikasämlinge der Sorte "Neusiedler Ideal Elite" wurden, nachdem sich 4 - 5 Blätter gut entwickelt hatten, mit einer wäßrigen Wirkstoffaufbereitung, die aus einer Stammlösung aus 10 % Wirkstoff, 63 % Cyclohexanon und 27 % Emulgiermittel angesetzt wurde, bis zur Tropfnässe besprüht. Am nächsten Tag wurden die behandelten Pflanzen mit einer Sporensuspension von *Botrytis cinerea,* die 1.7 x 10⁶ Sporen/ml in einer 2 %igen wäßrigen Biomalzlösung enthielt, inokuliert. Anschließend wurden die Versuchspflanzen in eine Klimakammer mit 22 bis 24°C und hoher Luftfeuchtigkeit gestellt. Nach 5 Tagen konnte das Ausmaß des Pilzbefall auf den Blättern visuell in % ermittelt werden.

In diesem Test zeigten die mit 250 ppm der erfindungsgemäßen Verbindungen I.13 und I.14 behandelten Pflanzen einen Befall von kleiner 40%, während die mit 250 ppm der Vergleichverbindungen B und C behandelten Pfanzen nahezu und die unbehandelten Pflanzen (Kontrolle) zu 100% befallen waren.

### 5) -Kurative Wirksamkeit gegen Puccinia recondita an Weizen (Weizenbraunrost)

Blätter von in Töpfen gewachsenen Weizensämlingen der Sorte "Kanzler" wurden mit Sporen des Braunrostes (*Puccinia recondita*) bestäubt. Danach wurden die Töpfe für 24 Stunden in eine Kammer mit hoher Luftfeuchtigkeit (90 bis 95 %) und 20 bis 22° C gestellt. Während dieser Zeit keimten die Sporen aus und die Keimschläuche drangen in das Blattgewebe ein. Die infizierten Pflanzen wurden am nächsten Tag mit einer wäßrigen Wirkstoffaufbereitung, die aus einer Stammlösung bestehend aus 10 % Wirkstoff, 63 % Cyclohexanon und 27 % Emulgiermittel angesetzt worden war, tropfnaß besprüht. Nach dem Antrocknen des Spritzbelages wurden die Versuchspflanzen im Gewächshaus bei Temperaturen zwischen 20 und 22° C und 65 bis 70 % relativer Luftfeuchte für 7 Tage kultiviert. Dann wurde das Ausmaß der Rostpilzentwicklung auf den Blättern ermittelt.

In diesem Test zeigten die mit 16 ppm der erfindungsgemäßen Verbindung I.14 behandelten Pflanzen keinen Befall, während die mit 16 ppm der Vergleichverbindungen B behandelten Pfanzen zu 40% und die unbehandelten Pflanzen (Kontrolle) zu 80% befallen waren.

### Beispiele für die Wirkung gegen tierische Schädlinge

Die Wirkung der Verbindungen der allgemeinen Formel I gegen tierische Schädlinge ließ sich durch folgende Versuche zeigen:

### Die Wirkstoffe wurden

a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

## Patentansprüche

1. Verbindungen der allgemeinen Formel I 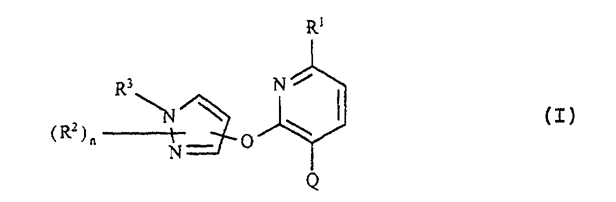 sowie deren Salze, in der die Substituenten und der Index n die folgenden Bedeutungen haben:
Q -C(=CHCH₃)-COOCH₃,
-C(=CHOCH₃)-COOCH₃,
-C(=NOCH₃)-COOCH₃ oder
-C(=NOCH₃)-CONH(CH₃);
R¹ Wasserstoff, Halogen, C₁-C₄-Alkyl, C₁-C₂-Halogenalkyl, C₁-C₄-Alkoxy oder C₁-C₂-Halogenalkoxy;
R² Halogen, C₁-C₄-Alkyl, C₁-C₂-Halogenalkyl, C₁-C₄-Alkoxy;
n 0, 1 oder 2, wobei die Substituenten R² verschieden sein können, wenn n = 2 ist;
R³ Phenyl, Pyridyl oder Pyrimidyl, wobei der Phenyl-, Pyridyl- oder Pyrimidylrest einen oder, unabhängig von-einander, zwei oder drei der folgenden Substituenten tragen kann: Halogen, Cyano, C₁-C₄-Alkyl, C₁-C₂-Halogen-alkyl, C₁-C₄-Alkoxy, C₁-C₂-Halogenalkoxy oder C₁-C₄-Alkoxycarbonyl.

2. Verbindungen der allgemeinen Formel Ia 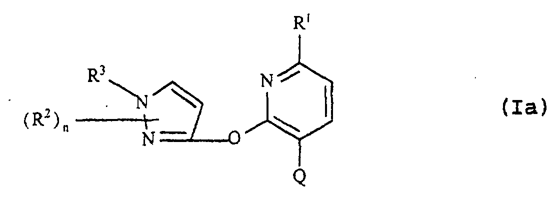 sowie deren Salze, in der der Substituent Q die in Anspruch 1 angegebene Bedeutung hat und
R¹ Wasserstoff, Halogen, Halogenmethyl;
R² Chlor, Brom, Methyl oder Trifluormethyl;
n 0 oder 1;
R³ Phenyl, wobei der Phenylrest einen oder, unabhängig von-einander, zwei oder drei der folgenden Substituenten tragen kann: Halogen, Cyano, C₁-C₄-Alkyl, C₁-C₂-Halogen-alkyl, C₁-C₄-Alkoxy, C₁-C₂-Halogenalkoxy oder C₁-C₄-Alkoxycarbonyl
bedeuten.

3. Verbindungen der allgemeinen Formel Ib 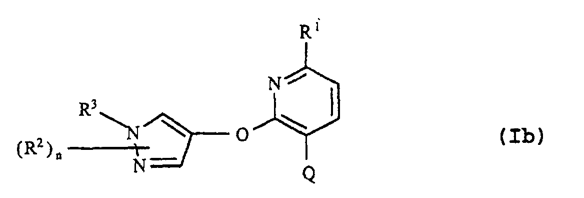 sowie deren Salze, in der der Substituent Q die in Anspruch 1 angegebene Bedeutung hat und
R¹ Wasserstoff, Halogen, Halogenmethyl;
R² Chlor, Brom, Methyl oder Trifluormethyl;
n 0 oder 1;
R³ Phenyl, wobei der Phenylrest einen oder, unabhängig voneinander, zwei oder drei der folgenden Substituenten tragen kann: Halogen, Cyano, C₁-C₄-Alkyl, C₁-C₂-Halogenalkyl, C₁-C₄-Alkoxy, C₁-C₂-Halogenalkoxy oder C₁-C₄-Alkoxycarbonyl
bedeuten.

4. Zur Bekämpfung von Schadpilzen und tierischen Schädlingen geeignetes Mittel, enthaltend eine wirksame Menge einer Verbindung der allgemeinen Formel I oder eines ihrer Salze gemäß Anspruch 1 und mindestens ein Formulierungshilfsmittel.

5. Verwendung der Verbindungen I oder ihrer Salze gemäß Anspruch 1 zur Herstellung von Mitteln gegen Schadpilze und tierische Schädlinge für den Pflanzenschutz.

6. Verfahren zur Bekämpfung von Schadpilzen und tierischen Schädlingen, **dadurch gekennzeichnet, daß** man die Schadpilze, deren Lebensraum oder die von ihnen freizuhaltenden Pflanzen, Flächen, Materialien oder Räume mit einer wirksamen Menge einer Verbindung der Formel I oder eines ihrer Salze gemäß Anspruch 1 behandelt.

## Claims

1. A compound of the formula 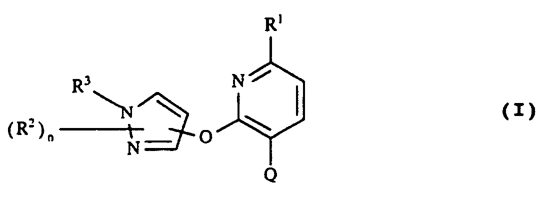 and salts thereof, in which the substituents and the index n have the following meanings:
Q is -C(=CHCH₃)-COOCH₃,
-C(=CHOCH₃)-COOCH₃,
-C(=NOCH₃)-COOCH₃ or
-C(=NOCH₃)-CONH(CH₃);
R¹ is hydrogen, halogen, C₁-C₄-alkyl, C₁-C₂-haloalkyl, C₁-C₄-alkoxy or C₁-C₂-haloalkoxy;
R² is halogen, C₁-C₄-alkyl, C₁-C₂-haloalkyl, C₁-C₄-alkoxy;
n is 0, 1 or 2, where the substituents R² may be different if n = 2;
R³ is phenyl, pyridyl or pyrimidyl, where the phenyl, pyridyl or pyrimidyl radical may carry one or, independently of one another, two or three of the following substituents: halogen, cyano, C₁-C₄-alkyl, C₁-C₂-haloalkyl, C₁-C₄-alkoxy, C₁-C₂-haloalkoxy or C₁-C₄-alkoxycarbonyl.

2. A compound of the formula Ia 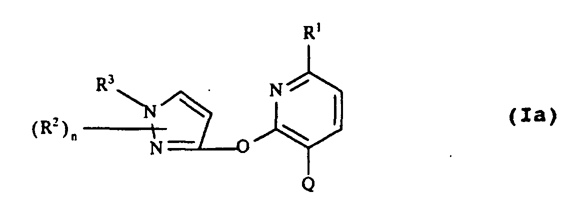 and salts thereof, in which the substituent Q is as defined in claim 1 and
R¹ is hydrogen, halogen, halomethyl;
R² is chlorine, bromine, methyl or trifluoromethyl;
n is 0 or 1;
R³ is phenyl, where the phenyl radical may carry one or, independently of one another, two or three of the following substituents: halogen, cyano, C₁-C₄-alkyl, C₁-C₂-haloalkyl, C₁-C₄-alkoxy, C₁-C₂-haloalkoxy or C1-C4-alkoxycarbonyl.

3. A compound of the formula Ib 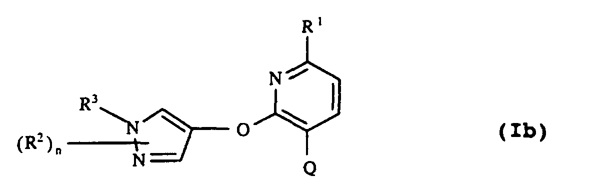 and salts thereof, in which the substituent Q is as defined in claim 1 and
R¹ is hydrogen, halogen, halomethyl;
R² is chlorine, bromine, methyl or trifluoromethyl;
n is 0 or 1;
R³ is phenyl, where the phenyl radical may carry one or, independently of one another, two or three of the following substituents: halogen, cyano, C₁-C₄-alkyl, C₁-C₂-haloalkyl, C₁-C₄-alkoxy, C₁-C₂-haloalkoxy or C₁-C₄-alkoxycarbonyl.

4. A composition suitable for controlling harmful fungi and animal pests, comprising an effective amount of a compound of the formula I or a salt thereof as claimed in claim 1 and at least one formulation auxiliary.

5. The use of the compounds I or salts thereof as claimed in claim 1 for preparing compositions against harmful fungi and animal pests for crop protection.

6. A method for controlling harmful fungi and animal pests, which comprises treating the harmful fungi, their habitat or the plants, areas, materials or spaces to be kept free from them with an effective amount of a compound of the formula I or a salt thereof as claimed in claim 1.

## Revendications

1. Composés de formule générale I 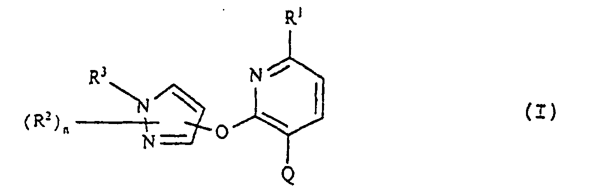 et leurs sels, les symboles et l'indice n ayant des significations suivantes:
Q -C(=CHCH₃)-COOCH₃,
-C(=CHOCH₃)-COOCH₃,
-C(=NOCH₃)-COOCH₃ ou
-C(=NOCH₃)-CONH(CH₃) ;
R¹ : l'hydrogène, un halogène, un groupe alkyle en C1-C4, halogénoalkyle en C1-C2, alcoxy en C1-C4 ou halogénoalcoxy en C1-C2 ;
R² : un halogène, un groupe alkyle en C1-C4, halogénoalkyle en C1-C2, alcoxy en C1-C4 ;
n : 0, 1 ou 2, les substituants R² pouvant être différents lorsque n = 2 ;
R³ : un groupe phényle, pyridyle ou pyrimidyle, chacun d'eux pouvant porter un ou, indépendamment les uns des autres, deux ou trois des substituants suivants : halogéno, cyano, alkyle en C1-C4, halogénoalkyle en C1-C2, alcoxy en C1-C4, halogénoalcoxy en C1-C2 ou (alcoxy en C1-C4)carbonyle.

2. Composés de formule générale Ia 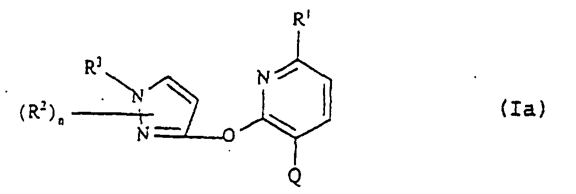 et leurs sels, le symbole Q ayant les significations indiquées dans la revendication 1, et les autres les significations suivantes :
R¹ : l'hydrogène, un halogène, un groupe halogénométhyle ;
R² : le chlore, le brome, un groupe méthyle ou trifluorométhyle ;
n : 0 ou 1 ;
R³ : un groupe phényle qui peut porter un ou bien, indépendamment les uns des autres, deux ou trois des substituants suivants : halogéno, cyano, alkyle en C1-C4, halogénoalkyle en C1-C2, alcoxy en C1-C4, halogénoalcoxy en C1-C2 ou (alcoxy en C1-C4)carbonyle.

3. Composés de formule générale Ib 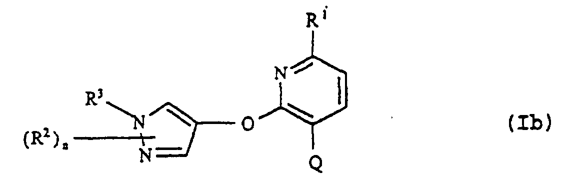 et leurs sels, le substituant Q ayant les significations indiquées dans la revendication 1 et les autres les significations suivantes :
R¹ : l'hydrogène, un halogène, un groupe halogénométhyle ;
R² : le chlore, le brome, un groupe méthyle ou trifluorométhyle ;
n : 0 ou 1 ;
R³ : un groupe phényle qui peut porter un ou bien, indépendamment les uns des autres, deux ou trois des substituants suivants : halogéno, cyano, alkyle en C1-C4, halogénoalkyle en C1-C2, alcoxy en C1-C4, halogénoalcoxy en C1-C2 ou (alcoxy en C1-C4)carbonyle.

4. Produit apte à l'utilisation pour la lutte contre les mycètes nuisibles et les parasites animaux, contenant une quantité efficace d'un composé de formule générale I ou de l'un de ses sels selon la revendication 1 et au moins un produit auxiliaire de formulation.

5. Utilisation des composés I ou de leurs sels selon la revendication 1 pour la préparation de produits aptes à l'utilisation pour la lutte contre les mycètes nuisibles et les parasites animaux dans la protection des végétaux.

6. Procédé pour combattre les mycètes nuisibles et les parasites animaux, **caractérisé par le fait que** l'on traite les mycètes nuisibles, leur habitat ou les végétaux, aires, matériaux ou locaux qu'on veut protéger contre les mycètes nuisibles par une quantité efficace d'un composé de formule I ou de l'un de ses sels selon la revendication 1.
